# EUROPEAN PATENT APPLICATION

(11) **EP 4 497 326 A1**
(43) Date of publication of application: **29.01.2025**
(21) Application number: 23186651.8
(22) Date of filing: 20.07.2023
(51) Int. Cl.: A01N 27/00, A01N 31/08, A01N 33/12, A01N 37/36, A01N 43/90, A01P 1/00, A61K 8/41, A61Q 11/00, A61Q 11/02, C11D 3/16, C11D 3/48

(54) **AQUEOUS SOLUTION WITH ANTI-MICROBIAL PROPERTIES, ASSOCIATED CLEANSING AGENTS AND SPECIFIC USE CASE**

(71) Applicant: Bottmedical AG, 4057 Basel (CH); Durecon Projects B.V., 3404LJ IJsselstein (NL)
(72) Inventor: Töpper, Tino, 79115 Freiburg (DE); Sirejacob, Gino, 9940 Evergem (BE)
(74) Representative: Mertzlufft-Paufler, Cornelius

(57) **Abstract**

An aqueous solution is proposed, containing an antimicrobial agent, a quaternary ammonium compound (QAC), an emulsifier, and at least one silane. Due to a build-up of a nanoscale-layer that provides the surface treated with the solution with antimicrobial-, antiviral-, deep-cleaning-, and anti-adhesive functionality, this solution is particularly effective in protecting surfaces against the formation of hazardous biofilms. The aqueous solution may thus be used for treating oral appliances, as a mouth wash, for disinfecting surfaces of medical devices, or as a sanitizer.

## Description

The present disclosure relates to an aqueous solution with anti-microbial properties. This solution may be used for cleaning and/or disinfecting surfaces, in particular for protecting surfaces against the build-up of a hazardous biofilm of microorganisms.

In various applications, ranging from medical applications to everyday life situations, there is a need for an efficient way of cleaning and disinfecting a surface. This may be particularly challenging, if the surface to be cleaned shows micro-pores, which are known to be ideal places for the cultivation of hazardous microorganisms such as bacteria, which tend to hide in the pores. In other words, such porous surfaces may be particularly hazardous as they constitute ideal growth places for microorganisms which are detrimental to human health.

For example, in the past, intraoral devices such as orthodontic devices or dental splints have been frequently plagued by the build-up of unwanted biofilms of hazardous microorganisms. Not only can such biofilms result in the production of unpleasant odor; they may also pose a threat to the health of the user of the device. For example, inflammations may result in the oral cavity from the microorganisms, which can result in serious damage to the gingiva and teeth. Especially when a dental appliance is worn purely for protection of teeth, the cleanliness and harmlessness of the device should be safeguarded.

Starting out from this background, it is an object of the invention to provide an aqueous solution that can be applied in a wide range of application cases, each time with the goal of reliably cleaning and/or disinfecting the surface to which the solution is applied. Another object of the invention is to achieve an antimicrobial protection, which is stable over longer terms. These objectives are of course linked to the general goal of enhancing the safety from infections resulting from the presence of microorganisms.

In accordance with the present invention, an aqueous solution is provided according to claim 1, which solves the aforementioned problem. In particular, the invention proposes an aqueous solution as introduced at the beginning, which, in addition, is characterized in that the solution comprises the following ingredients: at least one antimicrobial agent; at least one quaternary ammonium compound (QAC) and/or lactic acid; at least one emulsifier; and at least one silane.

In case of using the solution as a mouth wash, or for treating/cleaning an intraoral device, the surface may be a polymer surface of the device or the surface of the teeth of the user, for example. In other application cases, the surface treated with the solution may be that of a medical device. In other words, the proposed solution can be used in many different applications for cleaning and/or disinfecting surfaces, in particular for "deep-cleaning" of porous surfaces comprising microscopic pores. The term deep-cleaning may be understood herein in that said pores are cleaned thoroughly from microorganisms settling therein.

The composition proposed for the aqueous solution may also form the basis of other products, in particular cleansing agents such as a gel or a toothpaste or a tablet. Such products may also be used for treating and cleaning surfaces, thereby benefiting from the technical advantages of the invention.

In the following, the advantages and technical effects provided by the proposed composition of the solution will be explained:
First, the combination of at least one antimicrobial agent and a quaternary ammonium compound provides excellent antimicrobial properties to the solution. The term "antimicrobial agent" may be understood here as being synonymous to "active substance" or "active ingredient", because the respective agent is mainly responsible for the antimicrobial and/or antiviral effect that is provided by the solution.

Quaternary ammonium compound (QACs) are cationic salts of organically substituted ammonium compounds (and thus may be understood as a non-ionic surfactant) and have a broad range of activity against microorganisms: QACs are effective against Gram-positive bacteria and also against Gram-negative bacteria, and (although less effectively) against bacterial spores.

QACs are sometimes classified as surfactants (surfactants are compounds that are typically amphiphilic, meaning they have a polar or hydrophilic, i.e., water-soluble, part and a nonpolar, i.e. hydrophobic or lipophilic, part), because they can decrease the surface tension. QACs can thus act as detergents, wetting agents, and emulsifiers. An emulsifier is a substance that stabilizes an emulsion by reducing the oil-water interfacial tension. Emulsifiers are a part of the broader group of surfactants. Emulsifiers that are more soluble in water, and conversely less soluble in oil, will generally form oil-in-water emulsions; emulsifiers are not to be confused with solubilizers, which are compounds that increase the solubility of a substance in a solvent, either by changing the properties of the solvent or by micelle formation.

QACs are also known to be efficient non-oxidizing disinfectants. Their mode of action is on the cell membrane which leads to cytoplasm leakage and cytoplasm coagulation through interaction with phospholipids.

An example of a QAC is benzalkonium chloride; examples of QACs that are particularly suited to be used in the solution are: quarternized coco amine ethoxylate, cocoalkylmethyl [polyoxyethylene(15)] ammonium chloride, or quaternary polydimethylsiloxane.

The at least one QAC contained in the solution is not only responsible for the strong antimicrobial protection provided by the solution; the at least one QAC also provides another important technical effect, namely the radicalization of the at least one silane, thus rendering the at least one silane reactive. The silanes are thus activated to couple to the surface treated with the solution.

The silanes can also react with each other, as they are radicalized by the QACs. However, by storing the solution in plastic bottles (thus avoiding free OH-groups on the inner surface of the bottle), a cross-connection inside the bottle containing the solution at an excessive ratio can be avoided, such that the solution can remain activated over prolonged shelf life. In fact, a certain degree of silanes cross-connecting to each other is actually helpful to form the desired functionalized nano-layer (see more details below), because the layer formation is improved with a covalently (pre-)bonded 3D network reacting to the surface treated with the solution.

The at least one QAC also provides a deep cleaning effect when the solution is applied to a surface, in particular to a surface containing microscale pores. It is well known that microorganisms such as bacteria tend to accumulate and grow in such micro-pores. The QAC can result in an effective removal of such microorganisms from the pores. Thereby a deep cleaning can be achieved, in particular inside of polymer materials as they are used for dental appliances (to name one possible application). Polymers that can be efficiently cleaned with the solution are, for example, cellulose acetate, cellulose acetate butyrate, PLA, chitosan or polyester, co-polyester, polycarbonate, thermoplastic polyurethane, polypropylene (PP), polyethylene (PE), polypropylene and polyethylene copolymers, acrylic, in particular PMMA, cyclic block copolymers, polyetheretherketone, polyamide, polyethylene terephthalate, polybutylene terephthalate, polyetherimide, polyethersulfone, polytrimethylene terephthalate or a combination thereof (e.g., a blend of at least two of the listed hard polymeric materials). For such polymers, the efficiency of the achievable deep cleaning, which is crucial for reliable antimicrobial protection, depends on the polarity of side groups, on the absorption rate of the polymer, on the functionalization of the polymer surface and on the porosity of the polymer network (typically defined by the molecular weight and degree of branching of the polymer chains).

The at least one QAC can thus result in an effective removal of a biofilm of microorganism that has grown inside of micro-pores distributed over a (in particular polymeric) surface to be cleaned with the solution.

The technical advantage of using silanes in the solution is that they can be hydrolysed in situ to generate silanol. One of the most important features of the chemistry and structure of silanols is the high acidity of the group, which means that they tend to form strong hydrogen bonds (covalent bonds) both to themselves (resulting in cross-linking) and to organic molecules containing suitable hydrogen bonding sites such as OH-groups. Once covalently bond to a surface, the formed layer of silanes can render the surface hydrophobic and anti-adhesive. This has the advantage that the deposition of microorganism on the now hydrophobic surface is made more difficult, such that the growth of such microorganisms (bacteria, fungi) on the surface can be slowed down significantly. The solution can thus provide a protection against adhesion and growth of new microorganisms on the surface.

In other words, the proposed aqueous solution can provide anti-adhesive properties to the surface to which it is applied, thanks to the silanes comprised in the solution. Thus, through the formation of protection layers and loading of the pores with antimicrobial substances, the safety from infections resulting from the presence of microorganisms can be enhanced.

One core idea of the invention is thus that the silanes contained in the solution enable the deposition of a stable functionalized antibacterial and/or antiviral layer (on a surface) formed from the solution in the form of an "artificial skin".

In particular, the antimicrobial agents contained in the solution may be coupled to such a functionalized layer of silanes. For example, it is possible to produce a nano-layer of fluorinated-silanes using a solution according to the invention. In other words, when fluoride is contained in the solution, a nano-layer of fluorinated-silanes can be built up on surfaces treated with the solution.

For example, such a functionalized antibacterial and/or antiviral layer may be formed on teeth, on the surface of an oral appliance to be worn in the oral cavity such as a dental splint, or on almost any other surface that provides some suitable bonding site. Through a deposition of the nano-sized layer on the surface as provided by the solution, the growth of microorganisms on the surface can be effectively prevented or at least significantly reduced or postponed. Moreover, the activity of viruses present on these surfaces may be significantly reduced for several hours.

We note at this point that, although the solution is soluble in water, the layer formed from the solution will be insoluble in water and it may be coupled to the respective surface (to which the solution is applied) by covalent bonds, due to the chemistry provided by the silanes contained in the solution and activated by the QACs. Therefore, the layer will remain attached to the surface in an aqueous ambient (such as the oral cavity) over several hours and will not be washed away by saliva or other aqueous solutions, which is another important technical advantage.

The main technical effects that the proposed solution can deliver are consequently as follows:
(1) thorough and deep-acting cleaning effect;
(2) effective formation of a covalently bonded nano-layer with hydrophobic and anti-adhesive properties;
(3) antiviral properties (in particular, when lactic acid is present in the solution); and
(4) antibacterial properties (through the combination of an antimicrobial agent and at least one QAC).

When the silanes couple to the surface treated with the solution, a covalently bonded antimicrobial nanometer-thin layer is formed as a sort of artificial skin, which, in addition to the active antimicrobial agent, is responsible for the achieved protection from infections (normally resulting from the presence of microorganisms).

In summary, the proposed solution can thus provide antimicrobial-, antiviral-, deep-cleaning-, and anti-adhesive functionality to the surface treated with the solution.

The aqueous solution proposed herein may therefore be used as a care and/or cleaning solution.

For example, according to a first use case, the solution may be applied to an oral cavity, including teeth and surrounding oral tissues, for providing an antimicrobial and/or antiviral effect in the mouth.

The solution can also be used, however, for washing and treating oral appliances to be worn in the oral cavity, such as dental splints or retainers or braces or the like, according to a second use case.

According to a third use case, the solution may be used for cleaning surfaces of objects such as medical devices. In particular, the solution may be used as a sanitizer or disinfectant.

As the solution can also possess virucidal activity, it strongly reduces the activity of viruses exposed to the solution, in particular viruses belonging to the family of coronaviruses such as SARS-CoV-2. In such a case, it is preferred if the solution comprises lactic acid and cinnamaldehyde as an antimicrobial agent, because this particular combination results in an excellent virucidal activity.

The aqueous solution presented so far can be further elaborated and implemented in various ways, which are described in the sub-claims and detailed in the following:
For example, as already mentioned, the solution may comprise lactic acid as an ingredient. Adding lactic acid can fulfill two basic functions:
When using lactic acid in combinations with certain antimicrobial agents, in particular cinnamaldehyde, the solution can produce an antiviral effect (virucidal activity of the solution). Accordingly, the solution can contain preferably a combination of two different antimicrobial agents, as described in greater detail below.

In addition, the lactic acid can activate the silanes contained in the solution in the sense that, in particular in an acidic medium, the silanes can be hydrolyzed in-situ (i.e. at the spot to which the solution is applied to ) thereby generating silanol. This activation by hydrolyzation is key for enabling covalent bonds between the nanoscale layer, formed by the solution, and the surface to which said solution / layer is applied to.

Another effect of the use of lactic acid is that it can dissolve plaque that has formed on the teeth, which is thus beneficial for good health of the oral cavity, as microorganisms tend to grow on/in plaque. Therefore, lactic acid can be an important ingredient in the solution, in particular when using the solution as a mouth wash.

When using cinnamaldehyde as an antimicrobial agent in the solution and having lactic acid comprised in the solution, it is most preferably when the lactic acid and cinnamaldehyde are contained in the solution in a weight ratio of: lactic acid:cinnamaldehyde ≥ 0.5; in particular: lactic acid:cinnamaldehyde ≥ 1.0. Such ratios result in a highly effective protection against viruses.

The at least one agent can preferably comprise or be an antimicrobial agent. In particular, it is suggested to make use of agents derived from a natural plant extract as the antimicrobial agent. Such plant extracts may be, in particular, essential oils.

The agent contained in the solution may thus be derived from a bio-based material, most preferably from an essential oil extracted directly from (non-fossil) plants. By this approach, agents such as cinnamaldehyde, lime, thymol, eugenol, linalool, carvacrol, nutmeg, pimenta berry, rosemary, petitgrain, coffee, or anise may be employed as an antimicrobial agent in the solution, to name a few.

The at least one silane contained in the solution may comprise an alkoxysilane and/or an ethoxy silane. These types of silanes are especially suited to be used in combination with the QAC contained in the solution. Although ethanol can be produced as a by-product during formation of the nano-layer, the amount of ethanol will be in the range of ppm and is therefore not hazardous.

The solution can also comprise at least one chelating agent; Use of a chelating agent in the solution is beneficial, because, chelating agents such as sodium glucoheptonate can generate complexes with proteins and iron. As a result, cell membranes of the microorganisms will be complexed and related microorganism or plaque or coloring can thus be easier removed from the surface treated with the solution.

Chelation is a type of bonding of ions and molecules to metal ions. It involves the formation or presence of two or more separate coordinate bonds between a polydentate (multiple bonded) ligand and a single central metal atom. These ligands are called chelating agents, chelants, chelators, or sequestering agents. The advantage of using these types of chelating agents in the solution is an excellent cleaning effect, in particular in pores which may be present on the surface treated with the solution. Other than with cleaning solutions based on chlorides, a roughening effect of the surface treated with the proposed solution is not observed, which is another advantage, because such roughening tends to make the surface more vulnerable to the settlement of further microorganisms. Most suitable for such application cases have been found to be glutamic acid N,N-diacetic acid and/or a tetrasodium salt and/or sodium glucoheptonate and/or sodium gluconate.

The above chelating agents also have a positive effect on the activity of biocides against bacteria and fungi. Chelates appear to remove calcium and magnesium ions present in the membrane of the bacterial cells, thereby increasing the permeability of the membrane to biocides. As a result, by adding at least one chelating agent to the solution, the antimicrobial effect of the solution is enhanced.

It is furthermore of advantage, if the solution comprises at least one non-ionic surfactant. Such a non-ionic surfactant may be a tenside, for example.

Of particular advantage is the addition of an alcohol ethoxylate and/or of an alkylpolyglucoside, because adding such ingredients to the solution results in rapid surface wetting (for example due to the dual structural aspect of ethoxylated alcohols, which contain a hydrophobic and hydrophilic component) and excellent degreasing properties, a relatively low rest alcohol content, low odor of the solution, and in particular a close ethoxylation (EO) range. Ethoxylation is a chemical reaction in which ethylene oxide is added to other organic compounds or a substrate. It is the most widely practiced alkoxylation, which involves the addition of epoxides to substrates. In the usual application, alcohols and phenols are converted into R(OC2H4)nOH where n ranges from 1 to 10. Such compounds are called alcohol ethoxylates. Alcohol ethoxylates are often converted to related species called ethoxysulfates. Alcohol ethoxylates and ethoxysulfates are surfactants, used widely in cosmetic and other commercial products; they offer good emulsifier effect for water in oil and for oil in water: The lower the EO grade, the better is the oil solubility; the higher the EO grade, the better is the water solubility. Moreover, ethoxylated alcohols are biodegradable.

Narrow-range ethoxylates (NREs) are fatty alcohol polyglycol ethers with a narrow homolog distribution and are known as efficient non-ionic surfactants. They can be produced industrially, for example, by the addition of ethylene oxide onto fatty alcohols in the presence of suitable catalysts (layer compounds which have been calcined or hydrophobized with fatty acids). It is also known that narrow-range surfactants like alcohol ethoxylates and propoxylates can be incorporated into alkyl ether sulfates or mixed with other anionic surfactants and that they exhibit beneficial properties such as reduced irritation to skin/eyes and lower free alcohol content.

The solution can also comprise a quaternary amine compound and/or laureate capreate. The advantage of using such ingredients in the solution is an excellent emulsification, along with antistatic properties (resulting in efficient deep cleaning, because electrostatic repulsion is avoided) and a strong disinfecting effect. Other suitable ingredient achieving similar effects are glyceryl esters and derivates of glyceryl esters; the best emulsion effect has been observed for PEG-40 hydrogenated castor oil.

The solution can also comprise at least one emulsifier. Particularly suitable to be used with the other ingredients in the solution are glyceryl esters and/or derivates of glyceryl esters and/or hydrocolloids and/or polysorbates and/or gums, such as Xanthan gum, guar gum locust bean, Arabic gum, scleroglucan, or agar agar. The technical benefit provides by adding gums to the solution is a stable emulsification process, a higher viscosity, and a clinging effect (resulting from electrostatic adhesion). In simple words, the nano-layer formed on the surface will be more stable over time.

It has also been found that the antimicrobial effect of the solution can be significantly boosted, when the solution comprises at least two different antimicrobial agents. Each of the agents alone would provide an antimicrobial effect; however, if the two different agents are selected from the following group of agents, namely: Limonene, Methyl Salicylate, Cinnamaldehyde, Trans-Anethole, Eucalyptol and/or Carvacrol, a synergistic effect can be achieved. In more detail, it has been found by extensive lab experiments, that combinations of these six different agents can provide synergistic hygienic and sanitary effects that can provide superior protection against the settlement and growth of potentially harmful microbials, in particular bacteria of the Streptococcus family, as compared to the use of only one of these agents. The underlying reason is that combinations of these agents can result both in large lag phases and efficient suppression of growth of bacteria. Preferably, the antimicrobial agents contained in the care solution may be exclusively chosen from the list presented above, i.e., the care solution may not contain any other antimicrobial agents except those comprised in said group.

Alternatively or additionally to the antimicrobials agents presented so far, a combination of one or more ethoxylated organic compounds may be part of the solution for providing antimicrobial protection, most preferably chosen from the following group: terpenes, terpenoids, phenylpropenes; of course, such ingredients may also be combined with one another;

We note at this point, that, preferably, if at least two different agents are comprised in the solution, these may be agents which have been synthetically produced. This is because in this case the purity of the agents will be higher, and having less impurities in the solution is beneficial for safeguarding the proper chemical working mechanism of the solution during buildup of the nano-layer.

With the exception of Carvacrol, the above five remaining antimicrobial agents are particularly well suited for treating oral devices featuring a core made of glycol modified Polyethylen-terephthalat (PETG), especially when using a soft cover or cap layer made from cellulose acetate butyrate (CAB). Therefore, the invention suggests, in particular, the use of the aqueous care solutions presented herein for treating / preparing such oral devices for oral use, as long as Carvacrol is not comprised in the solution. For other polymer material systems/applications, however, Carvacrol may be a suitable antimicrobial agent.

A first particularly well-working composition of the solution suggests that the solution comprises as agents, preferably exclusively, Limonene as a first agent and Methyl Salicylate as a second agent. It has been found by extensive experiments, that this particular combination of agents provides a minimum growth rate (for example measured in Joule/h using a microcalorimeter) of relevant bacteria such as Streptococcus mitis and Streptococcus mutans.

Another well-working embodiment of the solution suggests that the solution comprises as agents, preferably exclusively, Cinnamaldehyde as a first agent and Methyl Salicylate as a second agent. It has been found by extensive experiments, that this particular combination of agents provides a maximum lag phase (measured in hours) until a relevant growth can be observed for bacteria such as Streptococcus mitis and Streptococcus mutans.

Yet another well-working embodiment of the solution suggests that the solution comprises as agents, preferably exclusively, Limonene as a first agent, Methyl Salicylate as a second agent, and Cinnamaldehyde as a third agent. It has been found by extensive experiments, that this particular combination of agents provides synergistic effects resulting both in a small growth rate of bacteria and also in a long lag phase for microbials in general.

Yet another well-working embodiment of the solution suggests that the solution comprises as agents, preferably exclusively, Limonene as a first agent), Methyl Salicylate as a second agent, Cinnamaldehyde as a third agent, and Trans-Anethole as a fourth agent. It has been found by extensive experiments, that this particular combination of agents provides synergistic effects resulting both in a small growth rate of bacteria and also in a long lag phase for microbials in general.

Yet another well-working embodiment of the solution suggests that the solution comprises as agents, preferably exclusively, Limonene as a first agent, Methyl Salicylate as a second agent, Cinnamaldehyde as a third agent, Trans-Anethole as a fourth agent, and Eucalyptol as a fifth agent. It has been found by extensive experiments, that this particular combination of agents provides synergistic effects resulting both in a small growth rate of bacteria and also in a long lag phase for microbials in general.

Finally, it is also possible that the solution comprises all six different agents comprised in said group of agents, namely Limonene, Methyl Salicylate, Cinnamaldehyde, Trans-Anethole, Eucalyptol and Carvacrol.

The solution can also comprise at least one, preferably exclusively one, solubilizer selected from the following group: a fatty acid ester, a benzoic alcohol, a glycerol-based surfactant, Polyglyceryl-4 Laurate, and/or Polyglyceryl-6 Caprate, and PEG-40 hydrogenated castor oil. These types of solubilizers have been found to interact well with the agents proposed herein and to provide a relevant surfactant-effect, which is beneficial for distributing the agents evenly on the surfaces to be treated with the care solution, thereby maximizing the antimicrobial protection. In such cases, it is preferred if a volume ratio between a total amount of the agents (contained in the solution) Va and a volume of the solubilizer Vs (also contained in the solution) is: Va/Vs > 1:40. Such ratios will result in best performance of the solution.

According to the invention, it is of particular advantage to apply the aqueous solution disclosed herein to a surface for the purpose of cleaning and/or disinfecting the surface. Such an application may be done in various ways, for example by spraying the solution onto a surface, by applying it with a wipe, by immersing the surface in the solution, or by gurgling the solution in the mouth, to name a few.

In particular, the solution may thus be used as a mouth wash and/or for treating an oral appliance to be worn in the oral cavity and/or for disinfecting a medical device and/or for cleaning and/or disinfecting a surface comprising pores of micrometer size. In all such cases, the user can benefit from the properties provided by the solution, as detailed above.

The aqueous solution may also contain further ingredients, depending on desired additional effects to be achieved: For example, for whitening and remineralization of teeth, nano-hydroxyappetite (nHA) and/or calcium carbonate and/or fluoride may be added. To strengthen antibacterial effects, molecular iodine and/or xylithol may be added to the solution. If an nutrition shall be provided with the solution as an add-on, vitamins may be added. And for rebuilding the oral flora (i.e., healthy bacteria normally found in the oral cavity), probiotics, in particular lactobacillus reuteri from DSM 17938 and/or lactobacillus reuteri ATCC PTA 6475, may be added.

Preferred examples of the proposed aqueous solution will now be described in more detail, although the present invention is not limited to these examples: for those skilled in the art, it is obvious that further examples of the present invention may be obtained by combining features of one or more of the patent claims with each other and/or with one or more features of an example as described or illustrated herein.

Exemplary and preferred composition / ingredients of the solution may be derived from the following examples, in which the volume-percentages are provided:
Example 1: an aqueous solution comprising 250 ml of water; 600 ml of an emulsifier, preferably PG-4; and 30 ml of each of five respective essential oils containing the antimicrobial agents Limonene, Methyl Salicylate, Cinnamaldehyde, Trans-Anethole, and Eucalyptol, respectively (= 5x30 ml = 150 ml); lactic acid (10%), silanes (0.5%);
Example 2: an aqueous solution in the form of a concentrate, containing the following ingredients: a blend of etheric oils (7%) providing different antimicrobial agents; quaternary ammonium compounds (QACs) (2.5%); silanes (0.5%); ethoxylated alocohol (3%); complex builders (5%), lactic acid (20%), emulsifier PEG40 (5%); water (57%). This concentrate can be diluted, depending on the application, in the following ratios: 0.5%, 1.0%, 1.5%, 2.0%, 2.5%, 3.0%;

## Claims

1. **Aqueous solution** with anti-microbial properties, in particular to be used
- as a mouth wash and/or
- for treating oral appliances to be worn in the oral cavity and/or
- for cleaning and/or disinfecting surfaces,
**characterized in that** the solution comprises the following ingredients:
- at least one antimicrobial agent,
- at least one quaternary ammonium compound and/or lactic acid,
- at least one emulsifier, and
- at least one silane.

2. Solution according to claim 1, wherein the solution further comprises lactic acid.

3. Solution according to one of the preceding claims,
- wherein the at least one agent (2) comprises or is an antimicrobial agent (2), preferably derived from a natural plant extract, in particular from an essential oil.

4. Solution according to one of the preceding claims,
- wherein the at least one silane comprises an alkoxysilane and/or an ethoxysilane and/or
- wherein fluoride is contained in the solution (1) such that a nano-layer of fluorinated-silanes can be build up on surfaces treated with the solution (1).

5. Solution according to one of the preceding claims, wherein the solution further comprises at least one chelating agent, in particular
- glutamic acid N,N-diacetic acid and/or
- tetrasodium salt and/or
- sodium glucoheptonate and/or
- sodium gluconate.

6. Solution according to one of the preceding claims, wherein the solution further comprises at least one non-ionic surfactant, in particular
- an alcohol ethoxylate and/or
- an alkylpolyglucoside.

7. Solution according to one of the preceding claims, wherein the solution comprises
- a quaternary amine compound and/or
- laureate capreate.

8. Solution according to one of the preceding claims, wherein the solution further comprises as emulsifier
- glyceryl esters and/or
- derivates of glyceryl esters and/or
- hydrocolloids and/or
- polysorbates and/or
- gums such as Xanthan gum, guar gum locust bean, Arabic gum, scleroglucan, or agar agar.

9. Solution according to one of the preceding claims, wherein the solution comprises PEG-40 hydrogenated castor oil as an emulsifier.

10. Solution according to one of the preceding claims,
- wherein the solution comprises at least two different antimicrobial agents, with each agent providing an antimicrobial effect, and
- the at least two different agents are selected from the following group of agents: Limonene, Methyl Salicylate, Cinnamaldehyde, Trans-Anethole, Eucalyptol and/or Carvacrol.

11. Solution according to one of the preceding claims, wherein the solution comprises as agents, preferably exclusively, Limonene as a first agent and Methyl Salicylate as a second agent.

12. Solution according to one of the preceding claims, wherein the solution comprises as agents, preferably exclusively, Cinnamaldehyde as a first agent and Methyl Salicylate as a second agent.

13. Care solution according to one of the preceding claims, wherein the solution comprises as agents, preferably exclusively, Limonene as a first agent, Methyl Salicylate as a second agent, and Cinnamaldehyde as a third agent.

14. Care solution according to one of the preceding claims, wherein the solution comprises as agents, preferably exclusively, Limonene as a first agent, Methyl Salicylate as a second agent, Cinnamaldehyde as a third agent, and Trans-Anethole as a fourth agent.

15. Care solution according to one of the preceding claims, wherein the solution comprises as agents, preferably exclusively, Limonene as a first agent, Methyl Salicylate as a second agent, Cinnamaldehyde as a third agent, Trans-Anethole as a fourth agent, and Eucalyptol as a fifth agent.

16. Care solution according to claim 10, wherein the solution comprises all six different agents comprised in said group of agents, namely Limonene, Methyl Salicylate, Cinnamaldehyde, Trans-Anethole, Eucalyptol and Carvacrol.

17. Solution according to one of the preceding claims, wherein the solution comprises at least one, preferably exclusively one, solubilizer (3) selected from the following group: a fatty acid ester, a benzoic alcohol, a glycerol-based surfactant, Polyglyceryl-4 Laurate, and/or Polyglyceryl-6 Caprate,
- preferably wherein a volume ratio between a total amount of the agents Va and a volume of the solubilizer Vs contained in the solution (1), respectively, is: Va/Vs > 1:40.

18. Use of a solution according to one of the preceding claims, **characterized in that** the solution is applied to a surface for cleaning and/or disinfecting the surface, in particular wherein the solution is used
- as a mouth wash and/or
- for treating an oral appliance to be worn in the oral cavity and/or
- for disinfecting a medical device and/or
- for cleaning and/or disinfecting a surface comprising pores of micrometer size.

19. Cleansing agent, provided in the form of
- a gel or
- a toothpaste or
- a tablet, and
**characterized in that**
- the cleansing agent is based on an aqueous solution as defined by one of the claims 1 to 17.
